# EUROPÄISCHE PATENTSCHRIFT

(11) **EP 1 887 859 B1**
(45) Veröffentlichungstag und Bekanntmachung des Hinweises auf die Patenterteilung: **05.12.2012**
(21) Anmeldenummer: 06742372.3
(22) Anmeldetag: 17.05.2006
(51) Int. Cl.: A01K 67/027, C07K 14/475, C07K 14/52

(54) **TIERMODELL FÜR DAS HUMANE IMMUNSYSTEM, SOWIE VERFAHREN ZU DESSEN HERSTELLUNG**
ANIMAL MODEL FOR THE HUMAN IMMUNE SYSTEM, AND METHOD FOR PRODUCING THE SAME
MODELE ANIMAL POUR LE SYSTEME IMMUNITAIRE HUMAIN ET PROCEDE DE PRODUCTION DUDIT MODELE

(30) Priorität: 17.05.2005 DE 102005023342
(43) Veröffentlichungstag der Anmeldung: 20.02.2008
(73) Patentinhaber: Universität Leipzig, 04109 Leipzig (DE)
(72) Erfinder: EMMRICH, Frank, 04299 Leipzig (DE); KAMPRAD, Manja, 04299 Leipzig (DE); ACKERMANN, Manuela, 04299 Leipzig (DE)
(74) Vertreter: Stötter, Gerd
(86) Internationale Anmeldenummer: PCT/DE2006/000893
(87) Internationale Veröffentlichungsnummer: WO 2006/122545

(56) Entgegenhaltungen:
- WO-A-01/15521
- DE-C1- 4 337 007
- BONNET D ET AL: "Cytokine treatment or accessory cells are required to initiate engraftment of purified primitive human hematopoietic cells transplanted at limiting doses into NOD/SCID mice." BONE MARROW TRANSPLANTATION. FEB 1999, Bd. 23, Nr. 3, Februar 1999 (1999-02), Seiten 203-209, XP002394254 ISSN: 0268-3369
- CASHMAN J D ET AL: "HUMAN GROWTH FACTOR-ENHANCED REGENERATION OF TRANSPLANTABLE HUMAN HEMATOPOIETIC STEM CELLS IN NONOBESE DIABETIC/SEVERE COMBINED IMMUNODEFICIENT MICE" BLOOD, W.B. SAUNDERS, PHILADELPHIA, VA, US, Bd. 93, Nr. 2, 15. Januar 1999 (1999-01-15), Seiten 481-487, XP000973368 ISSN: 0006-4971
- LAPIDOT T ET AL: "CYTOKINE STIMULATION OF MULTILINEAGE HEMATOPOIESIS FROM IMMATURE HUMAN CELLS ENGRAFTED IN SCID MICE" SCIENCE, AMERICAN ASSOCIATION FOR THE ADVANCEMENT OF SCIENCE,, US, Bd. 255, Nr. 5048, 28. Februar 1992 (1992-02-28), Seiten 1137-1141, XP000573945 ISSN: 0036-8075
- NOBUYOSHI MASAHARU ET AL: "Arrest of human dendritic cells at the CD34-/CD4+/HLA-DR+ stage in the bone marrow of NOD/SCID-human chimeric mice" BLOOD, Bd. 97, Nr. 11, 1. Juni 2001 (2001-06-01), Seiten 3655-3657, XP002394255 ISSN: 0006-4971
- DAO M A ET AL: "Immunodeficient mice as models of human hematopoietic stem cell engraftment" CURRENT OPINION IN IMMUNOLOGY, CURRENT BIOLOGY LTD, Bd. 11, Nr. 5, 1. Oktober 1999 (1999-10-01), Seiten 532-537, XP004257581 ISSN: 0952-7915
- SHULTZ LEONARD D ET AL.: "Human lymphoid and myeloid cell development in NOD/LtSz-scid IL2R gamma null mice engrafted with mobilized human hemopoietic stem cells.", JOURNAL OF IMMUNOLOGY, vol. 174, 15 May 2005 (2005-05-15), pages 6477-6489,
- KERRE T ET AL.: 'Adapted NOD/SCID model supports development of phenotypically and functionally mature T cells from human umbilical cord blood CD34(+) cells.' Bd. 99, 2002, Seiten 1620 - 1626, XP003013993
- VORMOOR J ET AL.: 'Immature human cord blood progenitors engraft and proliferate to high levels in severe combined immunodeficient mice.' Bd. 83, 1994, Seiten 2489 - 2497, XP000574284
- KOLLMANN ET AL.: "Reconstitution of SCID mice with human lymphoid and myeloid cells after transplantation with human fetal bone marrow without the requirement for exogenous human cytokines", PNAS, vol. 91, 1994, pages 8032-8036,

## Beschreibung

Die vorliegende Erfindung betrifft ein Verfahren zur Etablierung eines funktionsfähigen adaptiven humanen Immunsystems in einem geeignetem Tiermodell. Das Tiermodell eignet sich z. B. für präklinische pharmakologische Studien und die Herstellung von humanen Antikörpern. Hierfür werden immundefiziente Mäuse benutzt, die kaum immunologische Reaktionen gegen die übertragenen Zellen entwickeln.

Grundlegende Strategien und Technologien einer medizinischen Therapie insbesondere aus dem Bereich der Regenerativen Medizin werden oft in tierischen Modellen (z.B. unter Verwendung von Nagern oder Huftieren) charakterisiert. Bei der Überführung so gewonnener Prinzipien in die klinische Anwendung am Menschen, ist es oft schwierig abzugrenzen, ob ein therapeutisches Produkt oder eine therapeutische Strategie für ein menschliches Target optimal ist oder nicht. So gibt es zwei Möglichkeiten: a) das humane Target ist in der klinischen Anwendung invalid oder b) das therapeutische Agens hat nicht dieselben funktionellen Eigenschaften. Dies ist speziell für Tumor- und Transplantationstherapien (u.a. Stammzelltransplantationen) und mit besonderem Augenmerk bei Immuntherapien, die speziesspezifische monoklonale oder polyklonale Antikörperpräparationen nutzen, relevant. Pharmazeutische Unternehmen sehen sich mit der Herausforderung konfrontiert, dass nur aus ca. 10% der in klinischen Studien getesteten Wirkstoffe ein neues zugelassenes Medikament hervorgeht. Zur Absicherung sowie der Akzeptanz einer neuen Technologie oder Arbeitsstrategie sind präklinische Modelle und Werkzeuge erforderlich, bei denen menschliche Zellen und Gewebe in einen möglichst komplexen systembiologischen Zusammenhang untersucht werden können. Immundefiziente Mäuse, in denen menschliche Zellen des Immunsystems miteinander reagieren ohne abgestoßen zu werden, können dafür eine Antwort darstellen, da sie den Vorteil eines Kleintiermodells mit einer besseren Korrelation zu den klinischen Gegebenheiten verknüpfen. Zwei Strategien zur Entwicklung entsprechender Tiermodelle bei Nagern wurden bisher dargestellt.

Die erste Strategie nutzt die Technologie zur Herstellung genetisch veränderter (*transgener, knock-out, knock-in*) Tiere, mittels der Einführung menschlicher Gene, durch Induktion spezifischer Mutationen oder den Ersatz eines murinen Gens durch das humane Homolog in somatischen oder Keimbahnzellen um genetische Veränderungen nachzuahmen bzw. um humane Antigene oder Targets zu exprimieren. Die Grenzen dieser Methode liegen neben den hohen Kosten, den geringen Erfolgsraten insbesondere in der limitierten Herstellung, meist einzelner, humaner Eigenschaften und somit deren Reaktivität mit meist tierischen Partnermolekülen sowie meist nicht identischen Genexpressionskontrollmechanismen. Daher wird diese Methode meist ausschließlich zur Charakterisierung des Potenzials von Wirkstoffen bezüglich des Wirkstoffmetabolismus und der Bestimmung der Toxizität oder zur Untersuchung der Aktivität eines einzelnen oder eines Sets von Targets genutzt (1;2).

Der zweite und komplexere Weg ist die Erzeugung von Chimären durch Xenotransplantation (3). Dies umfasst die Applikation von humanen Zellen oder Geweben in meist immundefiziente Tiere. Herausragende Wirtstiere zur Erzeugung eines humanen Immunsystem sind Mäusestämme, die mehrfache Defekte in der adaptiven Immunität aufweisen wie Rag2^{-/-}γ^{-/-} (4), BNX oder NOD/SCID B2m^{null} (5). Als ein Standardmodell zur Humanisierung dienen verschiedene Linien der NOD/SCID- (non-obese-diabetic/severe-combined-immuno-deficiency) Maus. Diese sind im Wesentlichen durch folgende immundefiziente Eigenschaften charakterisiert: kompletter Verlust von B- und T-Lymphozyten, reduzierte Anzahl von NK-Zellen, Defekte in der Differenzierung und Funktion von Antigen-präsentierenden Zellen und der Abwesenheit von zirkulierendem Komplement. Diese Mäuse sind empfänglicher für ionisierende Strahlung als der Wildtyp und weisen Defekte im DNA-Reparatursystem auf. Die Ausbildung von humanen einzelnen bzw. mehreren Linien der Hämatopoese in einem immundefizienten Tier ist nach Transplantation von humanen hämatopoetischen Stammzellen, differenzierten hämatopoetischen Zellen sowie lymphoiden Organen möglich. In Abhängigkeit vom genutzten Mausstamm und dessen Konditionierung sowie der Anzahl und der Quelle von hämatopoetsichen Stammzellen ist die Entwicklung von 0,1 bis 90% humaner CD45⁺ Zellen im peripheren Blut oder der Milz und dem Knochenmark der Tiere möglich.

Nachteilig ist bisher die extrem hohe Variabilität der Rekonstitution der Hämatopoese. Zudem werden einzelne humane Zelltypen unterschiedlich repräsentiert, abhängig vom Mausstamm und der Konditionierung der Tiere. Kennzeichnend ist für die NOD/SCID-Maus: Es wachsen bevorzugt und fast ausschließlich B-Zellen. Die humanen B-Lymphozyten zeigen die typischen Muster des Immunglobulin-Genrearrangements (6) und generieren ein diverses Immunglobulin-Repertoire nach Applikation von humanem fötalen, Nabelschnurblut oder adulten lymphoiden Progenitoren (7). Zirkulierende T-Zellen fehlen oder werden nur in äußerst geringem Umfang und zudem noch verzögert gebildet. Nur nach einer Vorbehandlung der Tiere mit rekombinanten Tumor-Nekrosis-Faktor und der Applikation von hohen Zellzahlen mononukleärer Zellen ist eine schnelle und starke Entwicklung von T-Lymphozyten sichtbar (8). Das humane Engraftment weist jedoch sehr hohe Varianzen innerhalb der Tiergruppe auf. Die Phänotypisierung der humanen T-Zellen zeigt, trotz der Variabilität, einen CD4/CD8-Quotienten von 1:1 bzw. 1:2 in Abhängigkeit von der verwendeten Stammzellquelle. Die Zellen weisen ein diverses T-Zell-Rezeptor-Repertoire auf. Vorläufige Funktionsanalysen dieser T-Zellen zeigen in In-vitro-Proliferationsstudien mit PHA oder IL-2, dass sie aktiviert werden können, allerdings nur auf einem sehr niedrigem Niveau. US 6,627,792 B2 nutzt Sektionen von Rippen-Knochengewebe von operierten Patienten, welches subkutan in den Peritonealraum appliziert wird, zur erfolgreichen Rekonstitution von T-Zellen. Deren Verfügbarkeit und ethische Bedenken limitieren den Einsatz. Nach intravenöser Injektion von Einzelzellsuspensionen dieses Knochengewebes ist der humane Chimärismus sehr niedrig und T-Zellen konnten nur schwerlich nachgewiesen werden. US 6,060,643 nutzt zur Rekonstitution in BNX-Mäusen humane hämatopoetische Zellen von G-CSF-mobilisiertem peripheren Blut (PB), fötales und adultes Knochenmark (FKM, AKM). Nur 17% bzw. 4,7% der Mäuse waren mit einer extrem hohen Variabilität nach Transplantation von PB bzw. AKM nach 6-8 Wochen chimär. Kein Chimärismus wurde nach Applikation von FKM erzielt. Weniger als 3% bzw. 15% der mit PB oder AKM bzw. FKM transplantierten Tiere zeigten ein Langzeitengraftment. Tiere, die PB erhielten, haben eine mittlere Überlebensrate von 25% nach 30 Tagen. Dies könnte darauf zurückzuführen sein, dass autoreaktive Zellen gebildet wurden oder eine regulatorisch aktive Zellpopulation nicht ausgeprägt wird. Klare Zeichen einer Graft-versus-Host-Disease konnten nur nach Applikation von peripherem Blut adulter Spender gefunden werden (9). Dies zeigt die bisher unbefriedigende funktionelle Potenz von menschlichen Zellen im Kontext des Tiermodells an.

Humane Zellen der Makrophagen-Dendritischen Zellreihe konnten bisher nur als unreife CD34⁻ HLA-DR⁺ CD4⁺ Dendritische Zellvorläufer (DC) nach Implantation von humanen mononukleären Zellen des Nabelschnurblutes nachgewiesen werden. In den lymphoiden Organen der NOD/SCID-Maus konnten keine reife DC's detektiert werden (10). Obgleich die Differenzierung der DC zu reifen Zellen im Tiermodell geblockt war, zeigten sich die unter In-vitro-Bedingungen gereiften DC als effiziente Stimulatoren in einer gemischten Leukozyten-Reaktion. Cravens et al. (11) demonstrierte die Entwicklung von humanen reifen und reaktionsfähigen DC in der NOD/SCID-Maus ohne zusätzliche Zytokingabe. Die Zellen reagieren auf eine LPS-Stimulation *in-vivo* mit einer hohen Zytokinsekretion von IL-8, TNFα, IL-10 sowie IL-12p70. Relativ niedrig liegt die Freisetzung von IL-1ß, IL-6 sowie IFNγ. Diese Zytokine sind jedoch im humanen System charakteristisch für eine akute oder systemische Infektion. Weitere Entwicklungen sind daher nötig, in denen ein dem humanem System vergleichbares Zytokinprofil angestrebt werden muss.

Bonnet D et al. (Bone Marrow Transplant. 1999. 23(3):203-9) beschreiben die Transplantation von Lin⁻CD34⁺CD38⁻-Stammzellen aus dem Nabelschnurblut ("cord blood") oder "acute myeloid leukemia"-Stammzellen (AML) in NOD/SCID-Mäuse. Die Zugabe der Koloniestimulierende Faktoren oder eine Kotransplantation von Lin⁺CD34⁻ oder Lin⁻CD34⁺CD38⁺-Zellen als "accessory cells" ist notwendig für die erfolgreiche Transplantation der Stammzellen. Die verwendeten Hämatopoese- und Kolonie-stimulierenden Faktoren (CSF) sind Interleukin 3 (IL-3), Granulozyten/Makrophagen-CSF (GM-CSF), Granulozyten-CSF (G-CSF) u. der Stammzellen-Faktor (auch "Steel-Faktor" - SCF). Cashman J D et al (Blood. 1999. 93(2):481-7) beschreiben ebenfalls ein Transplantationsmodell für humane Stammzellen aus dem Nabelschnurblut in NOD/SCID-Mäuse. Hier erfolgt wieder die Zugabe von Kolonie-stimulierenden Faktoren, wie Stammzellen-Faktor (SCF oder "steel factor - SF), Erythropoetin (EP oder EPO), G-CSF, GM-CSF, IL-3, sowie Interleukin-6 (IL-6) und Flt3Ligand. Lapidot et al. (Science. 1992 255(5048):1137-41) beschreibt ebenfalls ein Transplantationsmodell für humane Knochenmarkzellen in SCID-Mäuse, welches nach Aussage der Autoren als Tiermodell der humanen akuten lymphoblastischen Leukämie dient. Nach der Transplantation erfolgt eine Stimulation der Mäuse durch die Applikation von humanen MGF (eine veraltete Bezeichnung des Stammzellen-Faktors SCF) und einem Fusionsprotein bestehend aus IL-3 und GM-CSF.

Vormoor et al. (Blood. 1994. 83(9):2489-97) beschreiben ein erfolgreiches Engraftment in 80% der SCID-Mäuse nach Injektion von Nabelschnurblutzellen, welches unabhängig von der Applikation von hämatopoetischen Zytokinen ist. Das multilineage Engraftment von durchschnittlich 10% humanen Zellen im Knochenmark wird von den Autoren als hoch eingeschätzt, welches dem damaligen Stand der Technik entsprach.

Kollmann et al. (Proc Natl Acad Sci USA. 1994.91(17):8032-6) zeigen ein signifikantes Engraftment humaner Zellen im Knochenmark und in der Peripherie nach Transplantation von *vorkultivierten* fötalen Knochenmarkzellen. Sie weisen B-Zellen und myeloide Zellen (Monozyten) nach, es wachsen keine T-Zellen aus.

Zwei jüngere Arbeiten konstatieren jeweils, dass die Funktion der (humanen) hämatopoetischen Stammzellen in NOD/SCID-Mäusen durch die noch vorhandene NK-Zellaktivität limitiert ist und verfolgen unterschiedliche Wege um diese Einschränkung zu überwinden. Kerre et al. (Blood. 2002 Mar 1;99(5):1620-6) optimieren die Humanisierung der NOD/SCID-Mäuse durch Blockierung der murinen NK-Zellaktivität mittels intraperitonealer Applikation von 200µg des Antikörpers TM-ß1. Shultz et al. (J Immunol. 2005. 174(10):6477-89) generieren einen neuen NOD/SCID-Mausstamm, in dem die γ-Kette des IL-2-Rezeptors defizient und somit die NK-Zellentwicklung blockiert ist. Als zusätzliche Optimierungsstrategie wurde die Applikation 20µg/Tier eines Fc-IL-7-Fusionsprotein verfolgt.

WO 01/15521 A1 offenbart eine Methode zur Generierung von transgenen Säugern, mit deren Hilfe die Herstellung einer spenderspezifischen Immunität erleichtert wird. Der Kern der Methode besteht in der Einführung von speziesspezifischen ausschließlich hämatopoetisch wirksamen Wachstumsfaktoren und Zytokinen (IL-7, SCF, LIF, GM-CSF, G-CSF, IL-6) als Transgen sowie der Inaktivierung von empfängerspezifischen Genen, die eine Immundefizienz des Empfängers gewährleisten.
DE 4337007 C1 offenbart ein Mausmodell zur Testung von AIDS-Impfstoffen, bei dem das Immunsystem von Mäusen durch Bestrahlung zerstört wird und anschließend embryonale Stammzellen transplantiert werden und die Mäuse mit IL-3 behandelt werden.

Zusammenfassend wird beurteilt, dass bisher existierende Verfahren und Modelle partiell die Entwicklung und Reifung von humanen hämatopoetischen Zellen und deren Reaktionsvermögen in einem Tier widerspiegeln. Bisher ist jedoch kein Verfahren etabliert, welches ein standardisiertes, reproduzierbares Modell generiert, dass die Gesamtheit eines humanen, insbesondere adaptiven Immunsystems repräsentiert. Dieses ist jedoch eine unbedingte Notwendigkeit um Testungen in vorklinischen Phasen effizienter zu machen und Therapieausfälle zu minimieren. Die Herstellung sowie die Verwendung von humanspezifischen therapeutischen oder regenerativen Agenzien setzt eine gemeinsame funktionelle Präsenz von humanen Dendritischen Zellen, T- sowie B-Zellen, NK-Zellen, Monozyten und Granulozyten voraus.

Die durch die Erfindung zu lösende Problemstellung ist daher, ein neues Verfahren zur dauerhaften Herstellung von human-spezifischen Chimären bereitzustellen, das nicht die aus dem Stand der Technik bekannten Nachteile aufweist. Insbesondere ist die Aufgabe der Erfindung, die Sicherheit des Anwachsens eines Transplantates mit Zellen, die ein hämatopoetisches Potential besitzen, in Tieren zu erhöhen und die interindividuelle Variabilität des Rekonstitutionserfolges zu minimieren. Damit wird die Voraussetzung von homogenen Versuchsgruppen mit vergleichbaren Reaktionsmuster und -stärke geschaffen.

Des Weiteren ist es Aufgabe der Erfindung, eine dauerhafte Ausbildung von allen Zellen des blutbildenden Systems in einem ausgewogenen Maße sowie die Ausprägung jeweils zelltypischer Reaktionsmuster in einem Tier zu erzielen. Die Erfindung soll die Kooperation der humanen Zellen in ihrer typischen Weise in einem Tier ermöglichen und so ein funktionsfähiges humanes, insbesondere adaptives Immunsystem darstellen. Die Chimären sollen keine oder nur minimale Anzeichen einer Transplantat-bedingten Reaktion gegen den Wirtsorganismus aufweisen. Zusätzlich soll die verfahrensbedingte, teilweise sehr hohe Sterblichkeit von Tieren (15-65%) minimiert werden.

Zur Lösung der Aufgabe wird mittels der Erfindung ein Verfahren bereitgestellt, das durch ein neues Konditionierungsverfahren der Wirtstiere, die bisherigen Nachteile der bekannten Lösungen überwindet.

Erfindungsgemäß wird die Aufgabe gelöst durch ein Verfahren zur Herstellung eines Tiermodells für das humane Immunsystem in einer immunodefizienten Maus, mit den Schritten:
a) Transplantation von humanen Stammzellen mit hämatopoetischen Potential in eine NOD/SCID-Maus und
b) Konditionieren der Maus mit Zellkulturüberstand einer Kultur von Zellen einer humanen Zelllinie ausgewählt aus 358/8 [abgeleitet aus NCI-H358] und daraus abgeleitete Subclonen, sowie A-549, wobei der Zellkulturüberstand jeweils 20 pg/ml bis 10 ng/ml der humanen Faktoren Interleukin-8, Interleukin-6, und vaskulo-endothelialen Wachstumsfaktor (VEGF), Interferon-γ-Induced Protein 10, Monocyte Chemoattractant Protein-1, Angiogenin und Tissue-type Plasminogen Activator enthält, und gegebenenfalls
c) Applikation von Stützzellen ohne eigenständiges hämatopoetisches Potential.

Die Erfindung wird charakterisiert durch die Nutzung von "konditionierten Zellkulturüberständen", die Kombinationen (Cocktails) von Botenstoffen insbesondere humanen Ursprungs enthalten. Diese werden in Verbindung mit der Applikation von humanen Stammzellen zur Herstellung des erfindungsgemäßen Tiermodells (Immunsystem-Chimären) in die immundefizienten NOD/SCID-Mäuse eingebracht.

Das erfindungsgemäße Verfahren ermöglicht vorteilhaft die Ausbildung sowohl von B- und T-Lymphozyten, NK-Zellen, Monozyten, Granulozyten, hämatopoetischen Stammzellen als auch von Dendritischen Zellen und die Ausprägung Zelltyp-spezifischer Reaktionen dieser Zellen. Damit steht ein Modell zur Verfügung, welches das humane, insbesondere das adaptive Immunsystem in wesentlichen Zügen seiner Funktionalität widerspiegelt. Das Verfahren ist für alle In-vivo- oder abgeleiteten In-vitro-Anwendungen nutzbringend, bei denen immunologische Prozesse mit menschlichen Zellen für diagnostische Zwecke, für Forschungszwecke, für die Entwicklung und Testung von Heilverfahren oder zur Entwicklung von Produkten benötigt werden. Beispielgebend sind hier die Herstellung und Verwendung von monoklonalen humanen Antikörpern, Entwicklung von Vakzinierungsstrategien, tierexperimenteller Krankheitsmodelle, Testung pharmakologischer Wirkstoffe, Entwicklung von Strategien zur Induktion von Immuntoleranz nach Organtransplantation insbesondere unter Verwendung humaner, humanisierter oder muriner Antikörper.

Die erfindungswesentlichen Merkmale des in den Patentansprüchen dargestellten Verfahrens sowie die in den weiteren Patentansprüchen geltend gemachten Ansprüche werden im Folgenden in verallgemeinerter Form erläutert.

Als erfindungswesentlich für die Erzeugung eines Chimärismus wird die Konditionierung mit "konditioniertem Zellkulturüberstand" in alleiniger oder kombinierter Verwendung mit Stützzellen bewertet.

Als "konditionierter Zellkulturüberstand" wird eine zellfreie, lösliche Phase definiert, die von Zellen abgetrennt wurde, die einzeln oder im Gewebeverbund vorliegen, nachdem während einer Inkubationsphase verschiedene Moleküle in den Zellkulturüberstand sezerniert wurden. Die sezernierenden Zellen entstammen derselben Spezies, wie die zur Erzeugung einer Chimäre genutzte Stammzellquelle. Es werden humane Zelllinien, die permanent oder nach geeigneter Stimulation bzw. Differenzierung lösliche Faktoren wie Zytokine produzieren, zur Herstellung des Zellkulturüberstands benutzt, welche aus den Zelllinien 358/8 (abgeleitet aus NCI-H358, European Collection of Cell Cultures, Salisbury, Wiltshire, UK, No. 95111733) und daraus abgeleitete Subklone (Subklon SC25, erhalten von Institut für Zoologie, Universität Leipzig), A-549 (humane TypII-Pneumozyten, LGC Promochem, Cell Biology Collection, ATCC^{®} No. CCL-185™, London, UK) sowie VA-ES-BJ (humane epitheloide Sarkomlinie, Deutsche Sammlung für Mikroorganismen und Zellkulturen GmbH, Braunschweig, DE, ACC 328) ausgewählt sind.

Der Zellkulturüberstand enthält die Faktoren, die im Zellkulturüberstand einer Zelllinie ausgewählt aus 358/8, A-549 und VA-ES-BJ oder in einem gemischten Zellkulturüberstand von zwei oder drei der genannten Zelllinien enthalten sind.

Als vorteilhaft hat sich erwiesen, wenn im Zellkulturüberstand die Faktoren IL-8, IL-6, VEGF, IP-10, MCP-1, ANG und tPA vorhanden sind.

IL-8, IL-6, VEGF, IP-10, MCP-1, ANG und tPA sind in den erwähnten Kulturüberständen in Konzentrationsbereichen von jeweils 20pg/ml bis 10ng/ml enthalten.

Besonders bevorzugt sind die Zytokine in den erwähnten Kulturüberständen in den nachfolgend ausgeführten Konzentrationsbereichen enthalten: IL-8 (500 pg/ml bis 10 ng/ml), IL-6 (50 pg/ml bis 1 ng/ml), VEGF (500 pg/ml bis 10 ng/ml), IP-10 (20 pg/ml bis 1 ng/ml), MCP-1 (500 pg/ml bis 10 ng/ml), ANG (20 pg/ml bis 1 ng/ml), und tPA (500 pg/ml bis 15 ng/ml).

Ein Gehalt an TNF-α, IL-10, MIG, RANTES, sVCAM-1, sCD40L, bFGF, sP-Selectin, oder IGF ist hingegen nicht oder nicht in höheren Konzentrationen (≤20pg/ml) notwendig.

Der Zeitpunkt dieser Konditionierung mit Zellkulturüberstand kann sowohl vor als auch nach dem Transplantationstag an einem oder mehreren Tagen erfolgen. Die Applikationsmenge sollte nicht den allgemeinen Empfehlungen, die in Richtlinien zur Behandlung von Versuchstieren verankert sind, zuwiderlaufen.

Der konditionierte Kulturüberstand wird bevorzugt unmittelbar nach der zellulären Transplantation appliziert und anschließend über einen Zeitraum von 14 Tagen jeden zweiten Tag verabreicht.

Der Applikationsort ist nicht beschränkt. Es empfiehlt sich jedoch eine intravenöse oder intraperitoneale Applikation. Für Mäuse sollte eine intraperitoneale Applikation mit einer Menge von ca. 200µl, bevorzugt 150 µl bis 250 µl pro Applikation, angestrebt werden. Es ist zweckmäßig, die Verträglichkeit jedes gewählten "konditionierten Zellkulturüberstandes" vor der Transplantation im Tiermodell zu überprüfen.

Zusätzlich zur Applikation der stammzellhaltigen Quelle werden in einer speziellen Variante des erfindungsgemäßen Verfahrens sogenannte "Stützzellen" ohne ein eigenständiges hämatopoetisches Potential appliziert werden. Dabei kann es sich z.B. um Stammzell-abgereicherte (maximaler Stammzellgehalt ≤ 0,1%) oder -negative Zellpopulationen (maximaler Stammzellgehalt ≤ 0,01%) einer Stammzell-Quelle als auch um mesenchymale Stammzellen handeln. Die Verwendung von "Stützzellen" mit onkogenem Potential sollte vermieden werden. Als Stützzellen können beispielsweise CD34-negative oder CD133-negative mononukleäre Zellen des Nabelschnurblutes oder des Knochenmark verwendet werden. Die Stützzellen werden bevorzugt in Konzentrationsbereichen von 1x10⁴ bis 5x10⁷ Zellen pro Zelltransplantat eingesetzt. Die Applikation der Stützzellen erfolgt bevorzugt gemeinsam mit der stammzellhaltigen Population.
Für das Tiermodell dienen NOD/SCID-Mäuseals Wirtstier zur Erzeugung des Chimärismus. Das Geschlecht der Tiere ist nebensächlich. Das Alter sollte insbesondere bei Mäusen mehr als 6 Wochen betragen. Das Verfahren wird erleichtert, da der Rezipient aus einer Inzuchtlinie stammt, die mehrere Immundefekte aufweist, vor allem in der adaptiven Immunantwort. Als bevorzugt wird hier der NOD/SCID Mausstamm des Jackson Laboratory, Bar Harbor, ME, USA benannt. Die immundefizienten Tiere sollten unter spezifisch-pathogenfreien Konditionen gehalten werden.

Zur Erzeugung des Chimärismus benötigte Stammzellen weisen ein hämatopoetisches (blutbildendes) Potential auf. Derartige hämatopoetische Stammzellen sind Zellen, die sich vermehren können und befähigt sind alle Zellen des blutbildenden Systems, insbesondere des Immunsystems, zu generieren. Solche Stammzellen lassen sich vor allem in Präparaten aus humanem Knochenmark, mobilisiertem sowie nicht-mobilisiertem peripherem Blut, Nabelschnurblut sowie -gewebe finden. Die erfindungsgemäß verwendeten Stammzellen sind in der Lage Zellen des humanen Immunsystems auszubilden. Insbesondere können die Stammzellen in B- und T-Lymphozyten, NK-Zellen, Monozyten, Granulozyten, sowie Dendritische Zellen differenzieren.

Das Alter und das Geschlecht der Spender sind unerheblich. Eine Anreicherung der Stammzellen ist möglich, aber nicht generell notwendig. Vorzugsweise sollte eine mononukleäre Einzelzellfraktion zur Verfügung gestellt werden. Die Entfernung der Erythrozyten sowie der Granulozyten durch gängige Methoden ist empfehlenswert. Sowohl frisch gewonnene als auch kryokonservierte Präparate können genutzt werden. Eine Zellfärbung mit ausgewählten Vitalfarbstoffen wie CFSE behindert nicht den Erfolg des Verfahrens.

Im Allgemeinen werden Tiere, in denen ein Chimärismus erzeugt werden soll, durch eine Konditionierung, die u.a. die Entfernung restlicher tierischer immunkompetenter Zellen verfolgt, für eine nachfolgende Transplantation der Stammzellen empfänglicher gemacht. Bevorzugt erfolgt dies durch eine radioaktive Bestrahlung des Empfängerorganismus. Als Strahlenquelle können radioaktive Isotope als auch Röntgengeräte genutzt werden. Die Bestrahlung sollte die jeweils tier-spezifische sub-lethale Dosen nicht überschreiten. Diese Dosis wird für jeden Zuchtstamm individuell ermittelt. In der Regel sollte eine einmalige und keine multiple Bestrahlung angestrebt werden. Die sub-lethale Dosis bei einer einmaligen Bestrahlung beträgt für NOD/SCID-Mäuse in der Regel 250-350 cGy.

Die Applikation des Transplantates kann kurz- oder langfristig nach der Konditionierung durch Bestrahlung erfolgen. Kurzfristig impliziert sofort nach der Bestrahlung, langfristig können mehrere Tage umschließen. Im Allgemeinen wird ein Zeitfenster zwischen 3 bis 24 Stunden nach der Bestrahlung berücksichtigt. Der Applikationsort des Transplantates ist nicht zwingend vorgeschrieben. Es sollte bei Einzelzellsuspensionen eine intravenöse oder intraperitoneale Gabe bevorzugt werden. Die Tiere können bei Bedarf anästhesiert werden. Bevorzugt werden pro Maus von 1x10³ bis 5x10⁷ mononukleäre Zellen, die die Stammzellen enthalten, transplantiert. Der Gehalt an CD34-positiven Stammzellen liegt vorzugsweise im Bereich von 5 bis 5x10⁵, bevorzugt 1x10³ bis 1x10⁴.

Nach der Transplantation werden die Tiere unter Standardbedingungen oder spezifisch-pathogenfreien Konditionen gehalten.

Die Ausprägung des erzeugten Chimärismus wird durch Kontrollen, in regelmäßigen Abständen, anhand von Proben des peripheren Blutes der transplantierten Tiere bestimmt. Bei Mäusen eignet sich besonders die Gewinnung von Blut aus dem retrobulbären Augenplexus oder aus der Schwanzvene. Der Anteil der humanen Zellen wird bevorzugt mittels durchflusszytometrischer Untersuchungen unter Verwendung von human-spezifischen Antikörpern (Linienmarker, Differenzierungsantigene, Aktivierungsmarker) ermittelt. Bei einem geringen Probenvolumen empfiehlt sich ein jeweils spezies-spezifischer pan-Leukozytenmarker wie CD45, möglichst im direkten Vergleich von Spender- und Empfängerspezies.

Vorteilhaft ist nach Anwendung des erfindungsgemäßen Verfahrens bereits 14 Tage nach Transplantation ein sicherer Nachweis von humanen Zellen in der Peripherie möglich. Diese Form des Monitoring erlaubt unter den Bedingungen der Erfmdung eine zuverlässige Aussage über den zeitlichen Rekonstitutionserfolg. Des Weiteren korreliert das Auftreten von humanen Zellen in der Peripherie mit einem Engraftment in der Milz und dem Knochenmark.

Die vorliegende Erfindung überwindet die Probleme und Nachteile des bisherigen Standes der Technik zur Herstellung human-chimärer Tiere, indem sie ein neues standardisierbares, hoch reproduzierbares Verfahren unter Verwendung eines neuen Konditionierungsverfahrens der Tiere in Verbindung mit humanen Stammzellen bereitstellt.

Das Verfahren erlaubt die Bereitstellung von gleichwertigen Chimären und schafft somit eine Reduktion der bisher nach dem Stand der Technik bekannten hohen interindividuellen Variabilität. Unter den Bedingungen der Erfindung ist nur eine Mortalitätsrate von 10% zu verzeichnen.

Ein neuer erfindungsgemäßer Aspekt ist die verlässliche Verwirklichung von Chimären, die humane immunkompetente Zellen des hämatopoetischen Systems bilden.

Das Tiermodell für das humane Immunsystem in einer immunodefizienten NOD/SCID-Maus, welches humane immunkompetente Zellen des hämatopoetischen Systems enthält, umfasst insbesondere T- und B-Zellen, Monozyten, Dendritische Zellen, NK-Zellen, Granulozyten sowie CD34⁺-Stammzellen. Alle Tiere weisen unter den Bedingungen der Erfindung ein humanes Engraftment im peripherem Blut, der Milz und dem Knochenmark auf. Vorteilhaft ist die dauerhafte Rekonstitution der Hämatopoese. Die Ausprägung sowie Zusammensetzung des menschlichen Blutbildes, die am Tag 56 nach der Transplantation in einer erfindungsgemäß behandelten NOD/SCID-Maus detektiert wurden, konnten vorteilhaft über einen Zeitraum von 5 Monaten nachgewiesen werden. Das erfindungsgemäß hergestellte Tiermodell für das humane Immunsystem ist daher sehr stabil.

Die Erfindung stellt vorteilhaft Mäuse zur Verfügung, die ein dem Menschen vergleichbares (äquivalentes) Immunsystem ausprägen, mit besonderem Fokus auf ein reaktives adaptives Immunsystem. Im erfindungsgemäß hergestellten Tiermodell haben die humanen immunkompetenten Zellen vorteilhaft eine vergleichbare Funktionalität, wie im menschlichen Körper, insbesondere eine vergleichbare Antwort auf Antigene und ein Zytokinprofil, welches dem humanen vergleichbar ist.

Die humanen dendritische Zellen in dem Tiermodell sind fähig, humanes Interleukin-8 konstitutiv und humanes Interleukin-6, humanes Interleukin-1ß und humanen Tumor-Necrose-Faktor-alpha (TNF-α) nach Immunstimulation (z. B. mit LPS) zu produzieren.

Erstaunlicherweise zeigen die Mäuse keine sichtbaren Zeichen einer Graft-versus-Host-Disease. Auf Grund der neuen, stabilen Ausprägung von humanen hämatopoetischen Stammzellen im Empfängerorganismus kann vorteilhaft eine weitere Transplantation von Zellen, die aus diesen stammen in einen oder mehrere sekundäre Rezipienten vorgenommen werden und somit eine weitere Vervielfältigung von chimären Organismen vorgenommen werden.

Die konditionierten, transplantierten Mäuse weisen in der Milz innerhalb der humanen Zellen in einem ausgewogenem Maße T-Zellen (Median: 29%) und B-Zellen (Median: 20%) auf. Des Weiteren können humane NK-Zellen (Median: 0,36%), Monozyten (Median: 1,1%), Granulozyten (Median: 4%) sowie CD34-positive Stammzellen (Median: 0,7%) detektiert werden. Der Median resultiert aus Untersuchungen mit 21 Tieren.

Die Bildung von humanem Immunglobulin M sowie Immunglobulin G *in vivo* als Beleg für die Aktivität der humanen B-Zellen konnte nachgewiesen werden.

Die hohe Reaktivität von Dendritischen Zellen (0,2% - 2,4% der humanen Zellen im Knochenmark) wurde durch Zytokinproduktion nach LPS-Stimulation *in vitro* gezeigt. Die Zellen produzieren sowohl 24 als auch 48 Stunden nach Stimulation Zytokine wie IL-1β (über 1000 pg/ml), IL-6 (über 5000 pg/ml), TNF-α (über 90 pg/ml) sowie IL-10 (über 100 pg/ml). Das Zytokin IL-8 wird bereits konstitutiv sezerniert. Dieses Zytokinmuster ist vergleichbar mit einem infektiösen Geschehen im Menschen. Mit der Reaktivität der Dendritischen Zellen als potente antigen-präsentierende Zellen (exogene als auch endogene Antigene), die eine Schlüsselposition bei der Aktivierung und Ausprägung einer adaptiven Immunantwort einnehmen, bei gleichzeitigem Vorhandensein von reaktiven humanen T- wie auch B-Zellen, ist eine dem menschlichen Immunsystem äquivalente Funktionalität des (chimären) Tiermodells gegeben.

Die Funktionalität des menschlichen adaptiven Immunsystems in der Maus konnte eindeutig mit dem Nachweis einer induzierten, T-Zellabhängigen, Antigen-spezifischen Antikörperproduktion (Immunisierung) gezeigt werden. Eine Reifung der Immunantwort in Form einer Bildung von Antigen-spezifischen Antikörpern des Immunglobulin G-Isotyps ist nachgewiesen.

Das erfindungsgemäß hergestellte Tiermodell stellt damit vorteilhaft ein Modellsystem für das humane Immunsystem dar, welches sich für die Produktentwicklung und Forschung, insbesondere pharmakologische Forschung, präklinische Studien, Pathogeneseforschung, Entwicklung von neuen diagnostischen oder therapeutischen Strategien eignet.

Das vorliegende Verfahren zur Herstellung von humanen reaktiven Chimären und das damit hergestellte Tiermodell erlaubt vorteilhaft die Herstellung humaner monoklonaler als auch polyklonaler Antikörper. Besonderer Stellenwert kommt der Herstellung kompletter humaner Antikörper ohne tierischen Fremdanteil und physiologischer vollständiger Glykosilierung zu. Diese humanen Antikörper haben einen hohen Stellenwert auf dem pharmazeutischen, therapeutischen sowie diagnostischen Markt.

Die erfindungsgemäß im Tiermodell generierten humanen Zellen haben vorteilhaft die Fähigkeit sowohl exogene als auch endogene Antigene zu erkennen und daraufhin eine Immunantwort auszubilden. Daraus leiten sich u.a. Möglichkeiten zur Herstellung von Antigen-spezifischen Dendritischen Zellen, Überprüfung immuntherapeutischer Protokolle z.B. unter Verwendung von humanen, humanisierten oder murinen therapeutischen Antikörpern, Charakterisierung von immunmodulatorischen Substanzen (Chromatinmodifikatoren, "small molecules") oder immunmodulatorisch wirksamer Zellpopulationen (mesenchymale Stammzellen, regulatorische T-Zellen, tissue engineered products) ab.

Dieses Modell kann vorteilhaft als eine Plattformtechnologie für die weitere Entwicklung von Modellen für Infektions- (wie HSV, EBV, HIV, Röteln), Autoimmun-, Tumor-, Transplantations-assoziierte oder entzündliche Erkrankungen und therapeutischer Strategien genutzt werden.

Im Folgenden wird das erfindungsgemäße Verfahren anhand eines Ausführungsbeispiels und den zugehörigen Abbildungen erläutert:

### 1. Gewinnung des konditionierten Überstandes

Eine humane Lungenzellkarzinom- (hier die TypII-Pneumozyten-Linie 358/8 [abgeleitet aus NCI-H358] Subklon SC25, erhalten von Institut für Zoologie, Universität Leipzig) wird unter sterilen Bedingungen in RPMI 1640 unter Zusatz von 5% fötalem Kälberserum kultiviert, bis sie die logarithmische Wachstumsphase erreicht. Zur Herstellung des konditionierten Mediums werden 2-4x10⁵ Zellen/ml ausgesät. Das Kulturmedium wird nach 3 Tagen geerntet, gesammelt, sterilfiltriert (Porengröße 0,2µm) und portioniert bei mindestens -20°C bis zur Verwendung gelagert.

In dem konditionierten Kulturüberstand sind die Zytokine in dem nachfolgend ausgeführten Konzentrationsbereichen enthalten: IL-8 (500 pg/ml bis 10 ng/ml), IL-6 (50 pg/ml bis 1 ng/ml), VEGF (500 pg/ml bis 10 ng/ml), IP-10 (20 pg/ml bis 1 ng/ml), MCP-1 (500 pg/ml bis 10 ng/ml), ANG (20 pg/ml bis 1 ng/ml), und tPA (500 pg/ml bis 15 ng/ml).

Ein Gehalt an TNF-α, IL-10, MIG, RANTES, sVCAM-1, sCD40L, bFGF, sP-Selectin, oder IGF ist hingegen nicht oder nicht in höheren Konzentrationen (≤20pg/ml) nachweisbar.

### 2. Methode zur Erzeugung des humanen Immunsystems in Mäusen

Mindestens 6 Wochen alte, männliche als auch weibliche NOD/SCID-Mäuse (Jackson Laboratories, USA) wurden unter spezifisch-pathogenfreien Bedingungen gehalten und dienten als Rezipienten. Die Ganzkörper-Bestrahlung der Tiere erfolgte mit 350cGy mittels eines Röntgentherapiegerätes. 3-5 Stunden nach der Bestrahlung erfolgte die Applikation von 1-10x10⁶ mononukleären Zellen, isoliert aus humanem Nabelschnurblut, in die Schwanzvene eines mittels Ether anästhesierten Tieres. Es wurden sowohl Zellen eines einzelnen Blutes eines Spenders als auch Zellen mehrerer, gemischter Blutproben genutzt. Die mononukleären Zellen wurden mittels einer Dichtegradientenzentrifugation unter Verwendung von Lymphozytentrennmedium LSM 1077 (PAA Laboratories, Pasching, AT) nach der Methode von Boyum (1976) isoliert. Die mononukleäre Zellfraktion wurde vor der Transplantation mindestens 24h bei -196°C gelagert und wurde am Tag der Transplantation zeitnah aufgetaut. Bis zur Transplantation erfolgte die Aufbewahrung der Zellen in Medium oder PBS bei 4°C. Nach der Applikation der Zellen erfolgte sofort die erste Gabe von 200µl des, wie oben beschrieben erhaltenen, konditioniertem Zellkulturüberstand der humanen Pneumozytenzelllinie intraperitoneal. Weitere Gaben des Überstandes erfolgten aller 2 Tage bis zum Tag 14 nach Transplantation, ohne eine Sedierung der Tiere vorzunehmen.

Um den Effekt der zusätzlichen Konditionierung zu demonstrieren, erhielten Vergleichsgruppen die jeweiligen Zellfraktionen ohne Applikation des Zellkulturüberstandes.

### 3. Charakterisierung des humanen Chimärismus sowie der Funktionalität des Immunsystems

Zur Kontrolle der humanen hämatopoetischen Rekonstitution der Mäuse wurden aller 2 Wochen, beginnend mit Tag 14 nach Transplantation, von jeder Maus durch Schwanzvenenpunktion oder leichtes Anritzen der Schwanzvene mit einem Skalpell, Blutproben von ca. 75µl gewonnen. Zur Vermeidung der Gerinnung wurde den Proben Natrium-Heparin zugefügt und anschließend wurden die Proben gut gemischt. 8 Wochen nach Transplantation wurden die Mäuse zur Bestimmung des humanen Langzeit-Engraftments getötet und das periphere Blut, die Milz, das Knochenmark sowie die Leber der Tiere isoliert. Von den soliden Organen wurden mechanisch Einzelzellsuspensionen hergestellt. Bei Bedarf erfolgte eine Passage über einen Nylonfilter zur Trennung von Bindegewebe von den Zellen. Erythrozyten wurden wahlweise mittels aqua dest. oder mit Ammoniumchlorid-haltigem Puffer lysiert. Zur Charakterisierung des Engraftments wurden direkt-konjugierte human-spezifische Antikörper verwendet, die vor der Analyse auf mögliche Kreuzreaktivität mit murinen Antigenen getestet wurden. Folgende human-spezifische Antikörper wurden genutzt: CD45, CD3, CD19, CD 56, CD14, CD16, CD34, HLA-DR, anti-lineage-cocktail, CD11c, CD123 (Becton Dickinson, Heidelberg, DE). Des Weiteren erfolgte zur Spezifitätskontrolle die Markierung mit einem murin-spezifischen CD45 Antikörper (IQ Products, Oldenburg, DE). Die Angabe der einzelnen Linien erfolgt als relativer Anteil von humanen CD45-positiven Zellen. Die markierten Proben wurden mittels einem FACS-Calibur (Becton Dickinson) analysiert. Rezipienten zeigten einen humanen Chimärismus, wenn sie einen Gehalt an humanen CD45-positiven Zellen von ≥ 0, 1 % aufwiesen.

Nach Transplantation von 5x10⁶ sowie 10x10⁶ mononukleären Zellen des Nabelschnurblutes unter Verwendung von konditioniertem Zellkulturüberstand zeigten alle Tiere einen humanen Chimärismus. Die Sterblichkeitsrate der Tiere lag bei 10%.

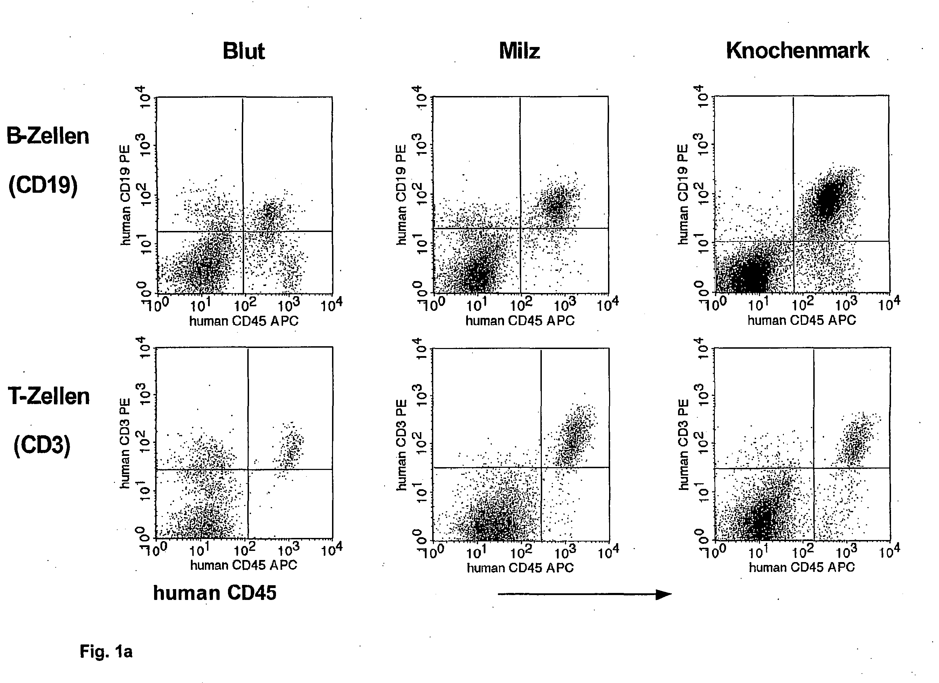

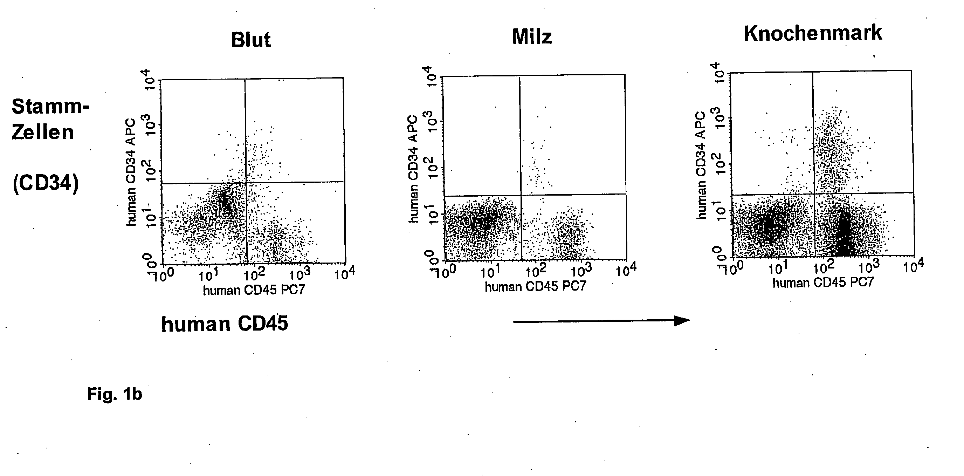

**Fig. 1** zeigt die Ergebnisse einer durchflußzytometrische Analyse von peripherem Blut, Milz und Knochenmark 8 Wochen nach Transplantation von transplantierten NOD/SCID-Tieren mit Applikation des konditionierten Überstandes. Die Dot Plots zeigen lebende Zellen charakterisiert durch human-spezifische Antikörper wie CD19 (allgemeiner B-Zellmarker), CD3 (allgemeiner T-Zellmarker), CD34 (Stammzellmarker, umfasst vor allem hämatopoetische Stammzellen sowie linien-geprägte Vorläuferzellen) aufgetragen gegen CD45 (allgemeiner Leukozytenmarker).

Wie in Fig. 1 dargestellt, konnten sowohl humane T-Lymphozyten als auch humane B-Lymphozyten und humane hämatopoetische Stammzellen generiert werden. Des Weiteren wurden humane Monozyten sowie humane NK-Zellen, als auch humane Granulozyten nachgewiesen. Die genannten Zellpopulationen lassen sich in allen hämatopoetischen Organen wie peripheres Blut, Milz und Knochenmark nachweisen.

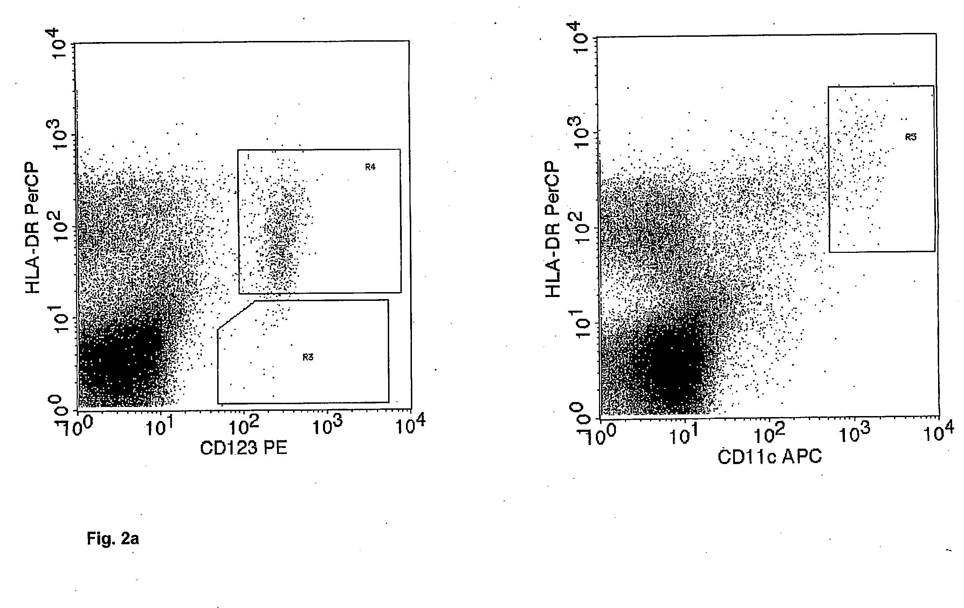

**Fig. 2a** zeigt eine durchflußzytometrische Analyse von Knochenmark (repräsentativ für Milz und peripheres Blut) 8 Wochen nach Transplantation von transplantierten NOD/SCID-Tieren mit Applikation des konditionierten Überstandes. Die Dot Plots zeigen humane Dendritische Zellen charakterisiert durch die Expression von human-spezifischen Antigenen wie HLA-DR, CD11c (rechter Blot, Region R5) oder CD123 (linker Blot, Region R4) sowie der Abwesenheit von linientypischen Antigenen wie CD3 (allgemeiner T-Zellmarker), CD16 (Marker von neutrophilen Granulozyten, Subpopulation von NK-Zellen und Monozyten, Makrophagen), CD19 und CD20 (allgemeine B-Zellmarker), CD14 (Monozytenmarker, Nachweis von Makrophagen, Neutrophilen, Eosinophilen) sowie CD56 (allgemeiner NK-Zellmarker, Subpopulation von T-Zellen) enthalten in einem anti-lineage-cocktail-Antikörper-Gemisch.

In Fig. 2a sind lebende Zellen dargestellt, die keine oder nur eine geringe Expression von linientypischen Antigenen zeigen. Diese Zellen enthalten zwei humane Dendritische Zellpopulationen.

Die plasmoide Dendritische Zellpopulation ist charakterisiert durch die Expression von CD123 sowie HLA-DR (Fig. 2a, linker Blot, Region R4). Im Knochenmark chimärer Tiere sind im Median 0,21% der Zellen plasmoide Dendritische Zellen. Nach einer systemischen Applikation von LPS (10µg/Maus), als Analogon einer systemischen bakteriellen Infektion, erhöht sich der Anteil der plasmoiden Dendritischen Zellen auf 2,4%.

Die myeloische Dendritische Zellpopulation exprimiert auf der Zelloberfläche CD11c sowie HLA-DR (Fig. 2a, rechter Blot, Region R5). Im Knochenmark der chimären Tiere sind im Median 0,17% der Zellen myeloische Dendritische Zellen. Nach einer systemischen Applikation von LPS (10µg/Maus), als Analogon einer systemischen bakteriellen Infektion, erhöht sich der Anteil der myeloischen Dendritischen Zellen auf 2,1%.

Dendritische Zellen sind ein wesentlicher Vertreter der Antigen-präsentierenden Zellen und bilden die Grundlage für die Ausbildung einer adaptiven Immunantwort. Des Weiteren sind sie wesentlich in den Prozess zur Entwicklung einer Immuntoleranz eingebunden. Die funktionelle Reaktivität der Zellen leitet sich aus der Erhöhung des Anteils Dendritischer Zellen nach einer systemischen Stimulation mit LPS ab.

Des Weiteren konnten humane basophile Zellen (Effektorzellen von allergischen TypI-Reaktionen) nachgewiesen werden (Fig. 2a, linker Blot, Region R3).

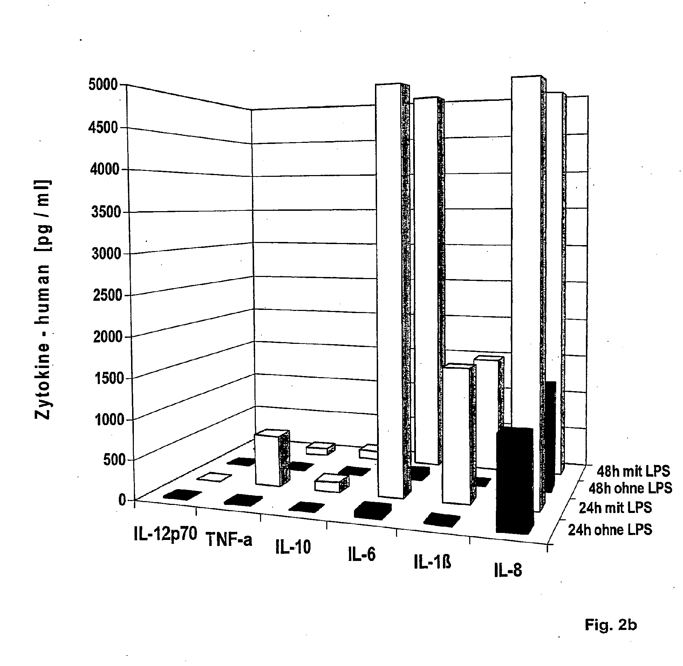

**Fig. 2b** vergleicht die Zytokinfreisetzung von isolierten Knochenmarkzellen nach LPS-Stimulation (10µg/ml, weiße Balken) von, unter Applikation des konditionierten Überstandes, humanisierten NOD/SCID-Mäusen im Vergleich zur unstimulierten Kontrolle (schwarze Balken).

Zur Aktivitätsprüfung der humanen Zellen wurden Knochenmarkzellen einer rekonstituierten NOD/SCID-Maus *in vitro* mit LPS (10µg/ml) als Analogon zu einer Infektion stimuliert. Kontrollen ohne Stimulation wurden mitgeführt. 24 bzw. 48 Stunden nach Stimulation erfolgte die Bestimmung human-spezifischer Zytokine wie IL-1β, IL-6, IL-8, IL-10, IL-12p70 sowie TNF-α in den Kulturüberständen mittels BD Cytometric Bead Array Analysis am FACS-Scan (Becton Dickinson).

Es konnte anhand von einer starken induzierbaren Zytokinfreisetzung klar die Reaktivität der humanen Dendritischen Zellen gezeigt werden (Fig. 2b). Die Zellen produzieren sowohl nach 24 als auch 48 Stunden nach Stimulation Zytokine wie IL-1β (über 1000 pg/ml), IL-6 (über 5000 pg/ml), TNV-α (über 90 pg/ml) sowie IL-10 (über 100 pg/ml). Das Zytokin IL-8 wird bereits konstitutiv sezerniert. Dieses Zytokinmuster ist vergleichbar mit einem Zytokinmuster bei einem infektiösen Geschehen im Menschen. Zudem kann auf Grund der fehlenden oder geringen Zytokinsekretion von IL-1β, IL-6, IL-10, TNF-α sowie IL-12p70 in den Kontrollkulturen konstatiert werden, dass die Zellen des Knochenmark keine Vorstimulation, die auf ein entzündliches Geschehen in der rekonstituierten Maus hinweist, aufzeigen.

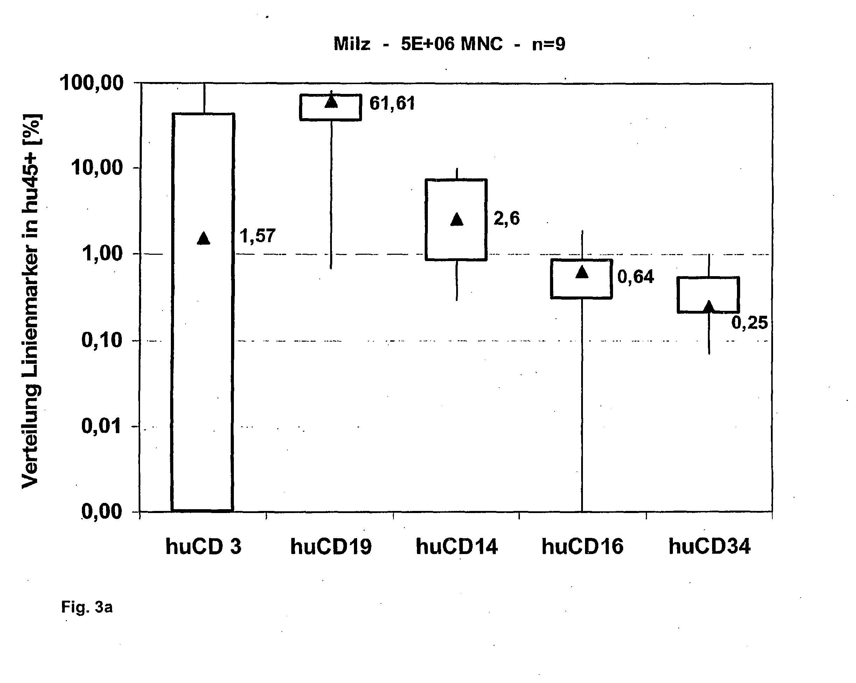

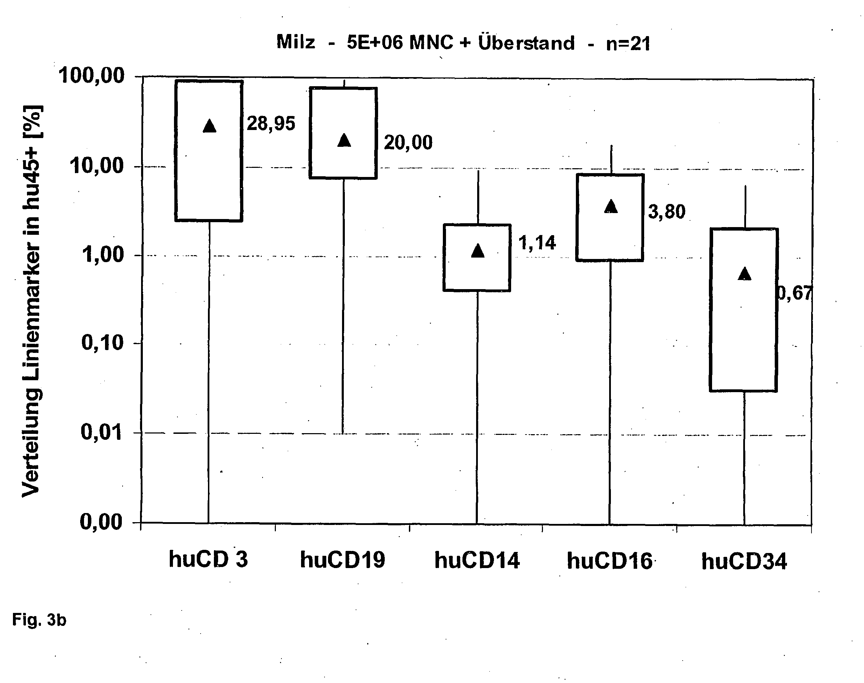

**Fig. 3a** und **3b** zeigen den Einfluss der Applikation des konditionierten Zellkulturüberstandes auf die Verteilung von humanen T-und B-Lymphozyten in der Milz von transplantierten Mäusen. Fig. 3a zeigt die Kontrolle ohne und Fig. 3b mit Applikation des konditionierten Überstandes. Die Angabe der einzelnen Zelllinien (Median, schwarze Dreiecke und Zahlen rechts neben Balken) erfolgt als relativer Anteil von humanen CD45-positiven Zellen. Die Balken stellen die Verteilung (2 Quartile sowie die beiden Extremwerte) innerhalb der Versuchstiere dar.

Vergleichend zur Kontrolle wird der Effekt der Konditionierung auf die Verteilung der T-sowie B-Lymphozyten in Fig. 3 verdeutlicht. Ohne Applikation des konditionierten Zellkulturüberstandes (Fig. 3a) bilden die humanisierten Tiere in überwiegendem Maße B-Zellen (Median: 62% CD19-positive Zellen) aus, die sich sowohl im Blut, als auch in der Milz und im Knochenmark nachweisen lassen. Im Median weisen diese Tiere nur 1,6% T-Zellen (CD3-positive Zellen) auf.

Nach Applikation des konditionierten Zellkulturüberstandes bilden (Fig. 3b) hingegen bilden sich in ausgewogenem Maße sowohl T-Zellen (29% CD3-positive Zellen) als auch B-Zellen (20% CD19-positive Zellen). Diese Tiere weisen eine mediane Verteilung von 1% Monozyten/Makrophagen (CD14-positive Zellen), 4% CD16-positive Zellen (NK-Zellen, Granulozyten, Subpopulation von Monozyten) sowie 0,7% CD34-positive Stammzellen bzw. geprägte Vorläuferzellen auf. Auf Grund der ausgewogenen Verteilung von B- sowie T-Lymphozyten ist die Voraussetzung gegeben, spezifische Immunreaktion insbesondere T-Zellabhängige Immunreaktionen auszubilden. Alle chimären Tiere bildeten nach Applikation des konditionierten Zellkulturüberstandes eine CD34-positive Stammzellpopulation aus, die insbesondere im Knochenmark lokalisiert werden kann. Damit ist eine dauerhafte Hämatopoese in den Tieren gewährleistet. Somit kann durch eine Transplantation des Knochenmarkes von ausgewählten chimären Tieren in weitere Rezipienten eine Vervielfältigung unter selektiven Bedingungen vorgenommen werden.

Mit dem Nachweis der einzelnen humanen Zelltypen (Fig. 1, 2, 3) sind die Voraussetzungen für die funktionelle Reaktivität insbesondere eines adaptiven Immunsystems gegeben.

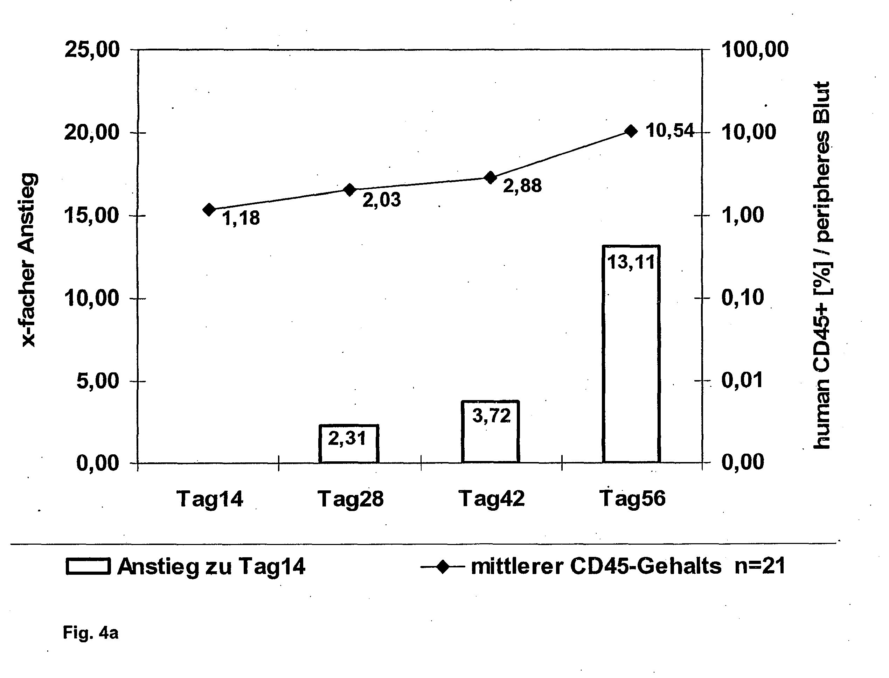

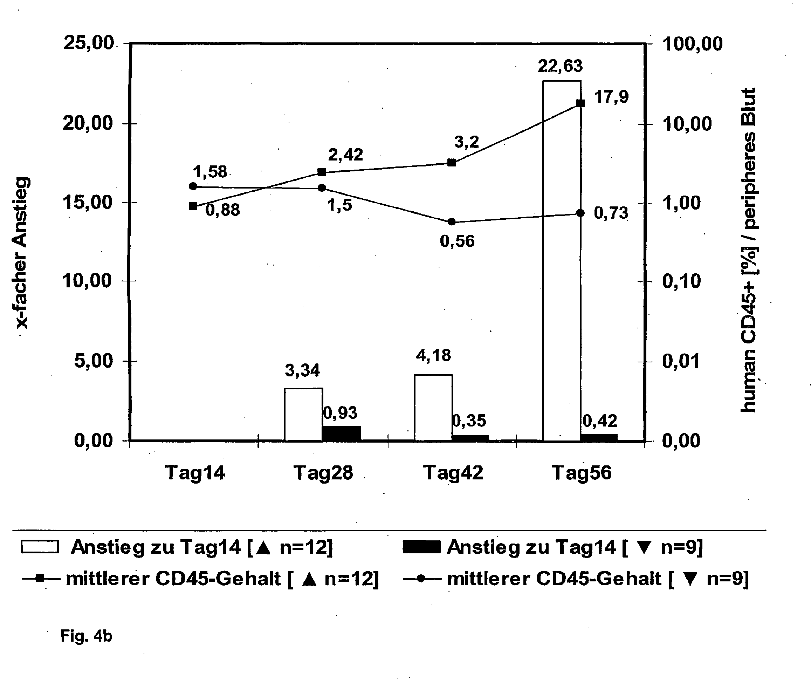

**Fig. 4a** **und** **4b** zeigen die Kinetik der Ausbildung eines humanen Chimärismus nach Transplantation von humanen mononukleären Zellen unter Verwendung des konditionierten Überstandes. Rezipienten, die einen Gehalt an humanen CD45-positiven Zellen von ≥ 0,1% aller Zellen zeigen, weisen einen humanen Chimärismus auf.

Dabei zeigt Fig. 4a den medianen Gehalt humaner CD45-positiver Zellen im Blut aller Tiere, die mit 5x10⁶ MNC transplantiert wurden (schwarze Linie mit Rauten), sowie den Anstieg des Gehaltes humaner CD45-positiver Zellen bezogen auf den Tag 14 (weiße Balken).

Fig. 4b zeigt den mittleren Gehalt humaner CD45-positiver Zellen im Blut der Tiere, die mit 5x10⁶ MNC transplantiert wurden, in Abhängigkeit von der Entwicklung des humanen Engraftments. Die Gruppierung der Tiere erfolgte in "low" Responder (Anstieg des relativen Anteils CD45-positiver Zellen <1 bezogen auf den Tag 14, n = 9 [9 Tiere]) sowie in "high" Responder (Anstieg des relativen Anteils CD45-positiver Zellen >1 bezogen auf den Tag 14, n = 12 [12 Tiere]).

Die Daten für die "low" Responder (Dreiecke mit Spitze nach unten) sind in der schwarzen Linie mit Kreisen und die schwarzen Balken angegeben. Die Daten für die "high" Responder (Dreiecke mit Spitze nach oben) sind in der schwarzen Linie mit Quadraten und die weißen Balken angegeben.

Das Oberflächenmolekül CD45 wird von allen Leukozyten exprimiert. Somit repräsentiert der Gehalt an humanen CD45-positiven Zellen die Entwicklung des humanen hämatopoetischen Systems im Wirt.

Alle Tiere zeigten nach Applikation von 5x10⁶ Zellen ein zu jedem Zeitpunkt nachweisbares humanes Engraftment (Gehalt an humanen CD45-positiven Zellen ≥ 0,1% aller Zellen). Wie in Fig. 4a dargestellt, steigt der Anteil CD45-positiver Zellen im Blut kontinuierlich vom Tag 14 nach der Transplantation bis zum Tag 56 an. Am Tag 56 ist im Mittel ein 13facher Anstieg zu verzeichnen. Der mittlere Gehalt humaner CD45-positiver Zellen im Blut aller transplantierten Tiere beträgt 10,5%. Es lässt sich jedoch innerhalb der Versuchstiergruppe eine Unterteilung in gute und schlechte Responder finden, die schon frühzeitig mittels der Bestimmung des CD45-Zellgehaltes festgelegt werden kann. Die Unterteilung erfolgte durch Bestimmung des Anstiegs des Gehaltes humaner CD45-positiver Zellen im Blut an allen weiteren Analysetagen im Verhältnis zum Tag 14. Ein Wert größer 1 defmiert "high" Responder, ein Wert kleiner 1 "low" Responder. So kann bereits am Tag 28, mit größerer Sicherheit am Tag 42 nach Transplantation eine klare Aussage über das Rekonstitutionsvermögen der einzelnen Tiere gemacht werden (Fig. 4b). "High" Responder weisen am Tag 56 nach der Transplantation im Mittel 18% humane CD45-positive Zellen im Blut auf, "low" Responder hingegen im Mittel nur 0,7%.

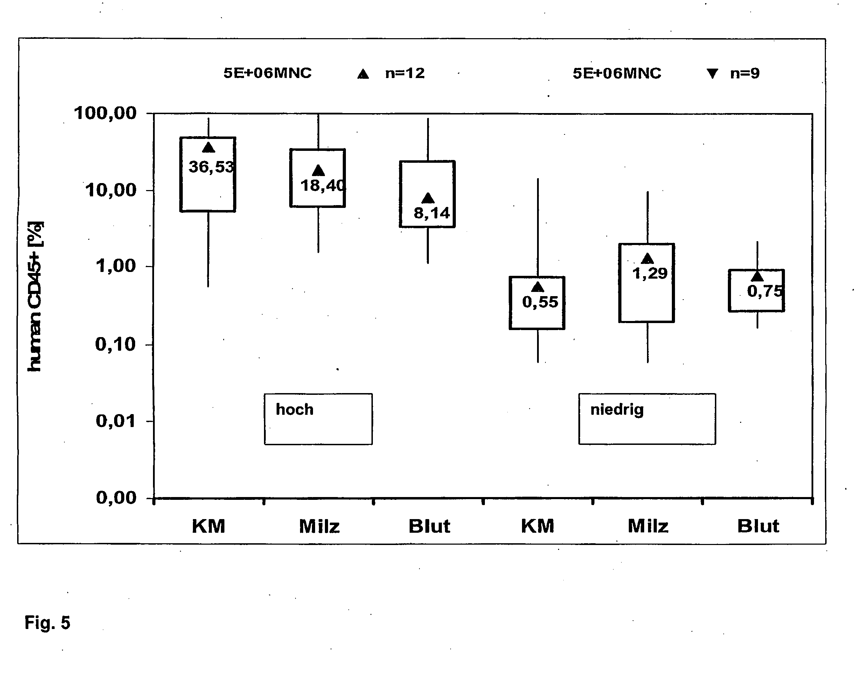

**Fig. 5** zeigt eine Darstellung des medianen relativen Gehaltes humaner CD45-positiver Zellen in lymphatischen Organen (Tag 56 nach Transplantation) von humanisierten Mäusen nach Applikation des konditionierten Überstandes in Abhängigkeit von einem niedrigen und hohen Engraftment. Die Gruppierung der Tiere erfolgte wie unter Fig. 4b beschrieben in "high"-Resonder (linke Seite in Fig. 5 - "hoch", n = 12 [12 Tiere]) und "low"-Responder (rechte Seite in Fig. 5 - "niedrig", n = 9 [9 Tiere]).

Die Bestimmung des relativen Gehaltes humaner CD45-positiver Zellen im peripheren Blut korreliert in hohem Maße mit dem Gehalt der humanen Zellen in der Milz sowie im Knochenmark (KM) (Fig. 5). So weisen "high" Responder im Median (schwarze Dreiecke In Fig. 5) 36,5% humane CD45-positive Zellen im Knochenmark auf, "low" Responder hingegen nur 0,6%. Vergleichbare Verhältnisse spiegeln sich in der Verteilung der Zellen in der Milz wider (18,4% versus 1,3%).

Somit lassen sich mit der Bestimmung des Anstiegs des Gehaltes humaner CD45-positiver Zellen im peripheren Blut frühzeitig homogene Versuchstiergruppen mit niedrigen interindividuellen Abweichungen für weiterführende Untersuchungen bilden. Die dargestellten Ergebnisse verdeutlichen zudem, dass alle hämatopoetischen Organe mit humanen Zellen besiedelt werden.

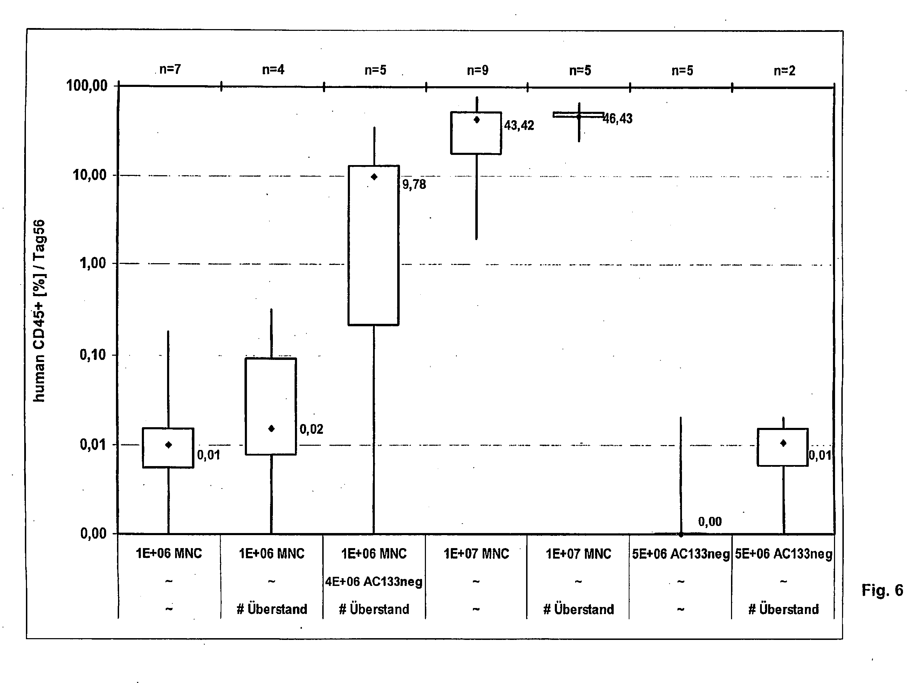

**Fig. 6** zeigt eine Darstellung des medianen relativen Gehaltes humaner CD45-positiver Zellen im Knochenmark von humanisierten Mäusen 8 Wochen nach Transplantation in Abhängigkeit von der applizierten Zahl von MNC und somit auch von darin enthaltenen Stammzellen, der Verwendung des konditionierten Überstandes sowie der Verwendung von Stützzellen. Der Median ist jeweils durch eine Raute und die zugehörigen Zahlenwerte rechts neben den Balken dargestellt. Als Stützzellen wurden AC133-negative Zellfraktionen der mononukleären Zellen des Nabelschnurblutes verwendet. AC133 ist ein Oberflächenmolekül, welches insbesondere von hämatopoetischen Stammzellen (Hauptanteil dieser Zellen trägt wiederum CD34) sowie von neuronalen Stammzellen und Vorläufern von Pankreaszellen exprimiert wird. Diese Zellfraktion wies in Kontrollexperimenten kein eigenes hämatopoetisches Engraftment auf.

Die Höhe des Chimärismus ist von der applizierten Zellzahl abhängig (Fig. 6). Nach Applikation von 1x10⁶ mononukleären Zellen (MNC) des Nabelschnurblutes ohne zusätzliche Konditionierung der Rezipienten mit konditioniertem Überstand prägen nur einige Tiere einen auf äußerst niedrigem Niveau liegenden Chimärismus aus (n = 7 [7 Tiere], linke Spalte). Im Median beträgt der relative Anteil humaner CD45-positiven Zellen 0,01% und liegt damit unterhalb der Definition eines erfolgreichen Engraftments.

Unter Verwendung des konditionierten Zellkulturüberstandes (n = 4 [4 Tiere], 2. Spalte von links) prägen die Tiere im Median 0,02% humane CD45-positive Zellen aus.

Erfolgt hingegen die Applikation der 1x10⁶ MNC mit 4x10⁶ Stützzellen (AC133-negative MNC des Nabelschnurblutes) und zusätzlicher Anwendung des konditionierten Überstandes können die Tiere erfolgreich einen humanen Chimärismus ausprägen (n = 5 [5 Tiere], 3. Spalte von links). Der mediane relative Anteil der CD45-positiven Zellen beträgt 9,8%.

Kontrolltiere, die 5x10⁶ Stützzellen (AC133-negative MNC des Nabelschnurblutes) erhielten, zeigten kein humanes Engraftment (n = 5 [5 Tiere], 2. Spalte von rechts). Eine zusätzliche Anwendung des konditionierten Überstandes unterstützte nicht die Ausbildung von humanen CD45-positiven Zellen (0,01% CD45-positive Zellen, n = 2 [2 Tiere,], rechte Spalte).

Bemerkenswert ist, dass nach Applikation von 10x10⁶ Zellen ohne Konditionierung mit Überstand ein deutlicher Chimärismus in den Tieren erzeugt werden konnte (43,4% CD45-positive Zellen, n = 9 [9 Tiere], mittlere Spalte).

Auffällig ist hingegen die extrem niedrige Variabilität des auf einem sehr hohen Niveau erzeugten Chimärismus nach Applikation von 10x10⁶ Zellen unter Konditionierung der Rezipienten mit Zellkulturüberstand (46,4% CD45-positive Zellen, n = 5 [5 Tiere], 3. Spalte von rechts). Diese niedrige Variablität in Verbindung mit einem stabilen, auf einem sehr hohen Niveau befindlichen humanen Chimärismus ist eine ideale Voraussetzung für weiterführende Anwendungen der humanisierten Maus (z.B. Pathogeneseforschung).

Zum Nachweis der Funktionsfähigkeit der gebildeten humanen B-Zellen in den Mäusen erfolgte die Bestimmung von humanem Immunglobulin M (IgM) bzw. Immunglobulin G (IgG) im Serum mittels ELISA. Der Test zeigt keine Kreuzreaktivitäten mit Maus-Immunglobulinen. Das Detektionslimit liegt bei 20ng/ml Immunglobulin.

Mittels human-spezifischer Bestimmung von Immunglobulinen (Ig) konnte eine In-vivo-Reaktivität von B-Zellen in den Chimären nach Transplantation von 5x10⁶ Zellen gezeigt werden. Eine IgG-Produktion konnte ab Tag 49 mit 752 ng/ml mit einem kontinuierlichen Anstieg bis auf 6728 ng/ml am Tag 56, begleitet von einer IgM-Produktion, detektiert werden.

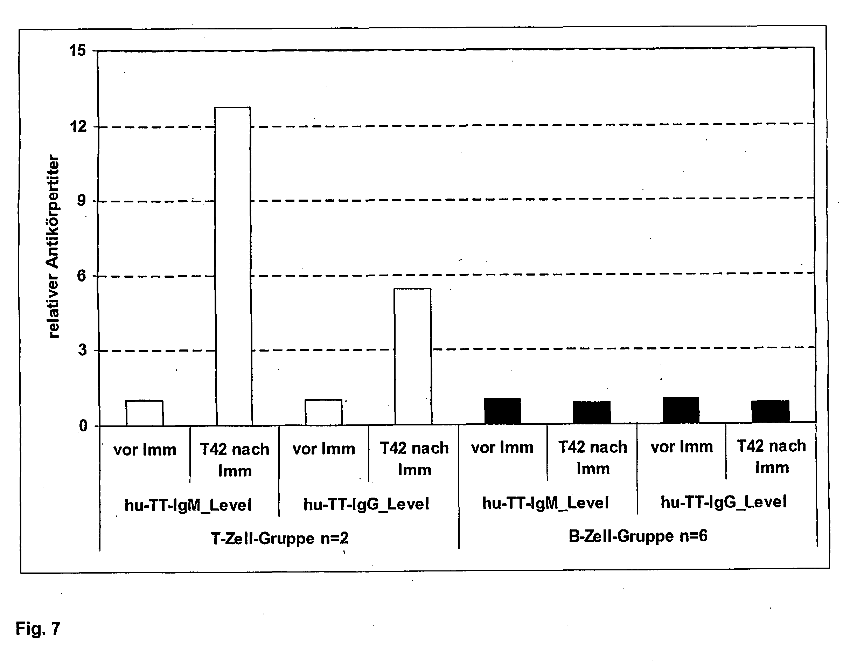

Zur Funktionsprüfung des humanen adaptiven Immunsystems wurde eine T-Zellabhängige Immunisierung der humanisierten NOD/SCID-Tiere mit Tetanustoxoid (TT) durchgeführt. Die Immunreaktivität wurde mit einem humanspezifischen, TT-spezifischen Antikörpernachweis (IgG, IgM) in einem ELISA, nachgewiesen (**Fig. 7**).

Das Experiment wurde in Tieren durchgeführt, die nach der dargelegten Methode ein komplettes menschliches Immunsystem mit einem relativen T-Zellanteil >20% (relativer Anteil CD3-positiver Zellen im Blut am Tag 56 nach Transplantation, n = 2) entwickelt hatten (T-Zellgruppe, links in Fig. 7). Als Kontrollgruppe dienten Tiere mit einem relativen humanen T-Zellanteil im Blut <2% (B-Zellgruppe, rechts in Fig. 7, n=6). Die Immunisierung erfolgte jeweils intra peritoneal am Tag 0 (Tag 0 der Immunisierung entspricht Tag 56 nach Transplantation), Tag 14, Tag 28, Tag 42 mit je 50 µg Tetanustoxoid in GERBU-Adjuvans 10 (Gerbu, Gaiberg). Die Antikörperbildung wurde am Tag 42 (T 42) nach der Immunisierung (entspricht Tag 112 nach der Transplantation) gemessen.

Fig. 7 zeigt, dass nach Immunisierung mit Tetanustoxoid (TT) in den Mäusen mit einem etablierten humanen Immunsystem eine Antigen-spezifische Immunantwort in Form von IgM- bzw. IgG-Antikörpern erfolgt. Bei den Tieren mit hohem T-Zellanteil (T-Zellgruppe -weiße Balken) lassen sich am Tag 42 (T42) nach der Immunisierung (Imm) Tetanustoxoidspezifische humane IgM (hu-TT-IgM) und IgG-Antikörper (hu-TT-IgG) nachweisen. Keine TT-spezifischen Antikörper wurden in den chimären Tieren mit einem niedrigen relativen T-Zellanteil (B-Zellgruppe - schwarze Balken) gebildet.

Das in den Mäusen erzeugte humane Immunsystem ist stabil und ist noch weit über 3 Monate nach Transplantation funktionsfähig, wie anhand einer T-Zell-abhängigen Immunantwort in Form von menschlichen Antikörpern nachgewiesen werden konnte (Fig. 7).

### Literaturverzeichnis:

(1) Tornell J, Snaith M. Transgenic systems in drug discovery: from target identification to humanized mice. Drug Discovery Today 2002; 7(8):461-470.
(2) Bolon B. Genetically engineered animals in drug discovery and development: A maturing resource for toxicologic research. Basic & Clinical Pharmacology & Toxicology 2004; 95(4):154-161.
(3) Igney FH, Asadullah K, Zollner TM. Techniques: Species' finest blend - humanized mouse models in inflammatory skin disease research. Trends in Pharmacological Sciences 2004; 25(10):543-549.
(4) Traggiai E, Chicha L, Mazzucchelli L, Bronz L, Piffaretti JC, Lanzavecchia A et al. Development of a human adaptive immune system in cord blood cell-transplanted mice. Science 2004; 304(5667):104-107.
(5) Kollet O, Peled A, Byk T, Ben Hur H, Greiner D, Shultz L et al. beta 2 microglobulindeficient (B2m(null)) NOD/SCID mice are excellent recipients for studying human stem cell function. Blood 2000; 95(10):3102-3105.
(6) Rossi MID, Medina KL, Garrett K, Kolar G, Comp PC, Shultz LD et al. Relatively normal human lymphopoiesis but rapid turnover of newly formed B cells in transplanted nonobese diabetic/SCID mice. Journal of Immunology 2001; 167(6):3033-3042.
(7) Kolar GR, Yokota T, Rossi MID, Nath SK, Capra JD. Human fetal, cord blood, and adult lymphocyte progenitors have similar potential for generating B cells with a diverse immunoglobulin repertoire. Blood 2004; 104(9):2981-2987.
(8) Samira S, Ferrand C, Peled A, Nagler A, Tovbin Y, Ben Hur H et al. Tumor Necrosis Factor Promotes Human T-Cell Development in Nonobese Diabetic/Severe Combined Immunodeficient Mice. Stem Cells 2004; 22(6):1085-1100.
(9) Vallet V, Mauray S, Kindler V, Aubry D, Ruegg M, Cherpillod J et al. Human tonsil implants xenotransplanted in SCID mice display broad lymphocytic diversity and cellular activation profile similar to those in the original lymphoid organ. Xenotransplantation 2005; 12(1):38-48.
(10) Nobuyoshi M, Kusunoki Y, Seyama T, Kodama K, Kimura A, Kyoizumi S. Arrest of human dendritic cells at the CD34(-)/CD4(+)/HLA-DR+ stage in the bone marrow of NOD/SCID-human chimeric mice. Blood 2001; 97(11):3655-3657.
(11) Cravens PD, Melkus MW, Padgett-Thomas A, Islas-Ohlmayer M, del PM, Garcia JV. Development and Activation of Human Dendritic Cells In Vivo in a Xenograft Model of Human Hematopoiesis. Stem Cells 2005; 23(2):264-278.

### Abkürzungsliste:

In der Erfindungsbeschreibung und in den Figuren werden folgende Abkürzungen verwendet:
- ANG: Angiogenin
- APC: Allophycocyanin
- bFGF: basic fibroblast growth factor
- CD: cluster of differentiation (engl.)
- CFSE: carboxyfluorescin succinimidyl ester (engl.)
- DSZM: Deutsche Sammlung von Mikroorganismen und Zellkulturen GmbH
- EBV: Epstein-Barr-Virus
- ELISA: enzym linked immuno sorbent assay (engl.)
- HLA: human leukocyte antigen
- HIV: human immunodeficiency virus
- HSV: human stomatitis virus
- hu: human
- IGF: insulin-like growth factor
- IL: Interleukin
- IP-10: interferon-γ-induced protein 10
- KM: Knochenmark
- LPS: Lipopolysaccharid
- MCP-1: monocyte chemoattractant protein-1
- MIG: monokine-induced by interferon-γ
- MNC: mononuclear cell (engl.)
- NOD: non-obese diabetic
- NK: Natürliche Killer (natural killer, engl.)
- PC7: Phycocyanin 7
- PE: Phycoerythrin
- PerCP: Peridinin chlorophyll protein
- RANTES: regulated upon activation, normal T-cell expressed and secreted
- sCD40L: soluble CD40-Ligand
- SCID: severe-combined-immuno-deficiency (engl.)
- sP-Selectin: Soluble platelet selectin
- sVCAM-1: vascular cellular adhesion molecule-1
- TNF-α: tumor necrosis factor-alpha
- tPA: tissue-type plasminogen activator
- VEGF: vascular endothelial growth factor

## Patentansprüche

1. Verfahren zur Herstellung eines Tiermodells für das humane Immunsystem in einer immunodefizienten Maus mit den Schritten:
a) Transplantation von humanen hämatopoetischen Stammzellen in eine NOD/SCID-Maus
und
b) Konditionieren der Maus mit Zellkulturüberstand einer Kultur von Zellen einer humanen Zelllinie ausgewählt aus 358/8, welche aus NCI-H358 abgeleitet ist, und daraus abgeleitete Subclonen, sowie A-549, wobei der Zellkulturüberstand jeweils 20 pg/ml bis 10 ng/ml der humanen Faktoren Interleukin-8, Interleukin-6, und vaskulo-endothelialen Wachstumsfaktor (VEGF), Interferon-γ-Induced Protein 10, Monocyte Chemoattractant Protein-1, Angiogenin und Tissue-type Plasminogen Activator enthält.

2. Verfahren nach Anspruch 1, **dadurch gekennzeichnet, dass** die humanen Stammzellen mit hämatopoetischen Potenzial aus humanem Knochenmark, mobilisiertem sowie nicht-mobilisiertem peripherem Blut, dem Nabelschnurblut oder Nabelschnurgewebe stammen.

3. Verfahren nach Anspruch 1 oder 2, **dadurch gekennzeichnet, dass** die Transplantation 3 bis 24 Stunden nach einer Bestrahlung der Maus erfolgt.

4. Verfahren nach einem der Ansprüche 1 bis 3, **dadurch gekennzeichnet, dass** die Konditionierung durch intravenöse oder intraperitoneale Applikation des Zellkulturüberstandes erfolgt.

5. Verfahren nach einem der Ansprüche 1 bis 4, **dadurch gekennzeichnet, dass** das Konditionieren in einem Zeitraum von 4 Wochen nach Transplantation, beginnend mit dem Tag der Transplantation, durchgeführt wird.

6. Verfahren nach einem der Ansprüche 1 bis 5, **dadurch gekennzeichnet, dass** zusätzlich zu dem Konditionieren mit Zellkulturüberstand eine Applikation von Stützzellen ohne eigenständiges hämatopoetisches Potenzial in die Maus erfolgt.

7. Verfahren nach einem der Ansprüche 1 bis 6, **dadurch gekennzeichnet, dass**, das Alter der Maus mehr als 6 Wochen beträgt.

## Claims

1. Method for preparing an animal model for the human immune system in a non-human mammal, comprising the steps of:
a) transplantation of human hematopoietic stem cells into a NOD/SCID mouse, and
b) conditioning the mouse with cell culture supernatant of a culture of a human cell line, selected from 358/8, which has been derived from NCI-H358, and sub clones thereof, as well as A-549, wherein the culture supernatant contains 20 pg/ml to10 ng/ml of each of the human factors interleukin-8, interleukin-6, vascular endothelial growth factor (VEGF), interferon-γ-Induced Protein 10, monocyte chemoattractant protein-1, angiogenin und tissue-type plasminogen activator.

2. Method according to claim 1, **characterized in that** the human stem cells having hematopoietic potential are derived from human bone marrow, mobilized or non-mobilized peripheral blood, umbilical cord blood or umbilical cord tissue.

3. Method according to claim 1 or 2, **characterized in that** the transplantation is carried out 3 to 24 hours after irradiation of the mouse.

4. Method according to one of the claims 1 to 3, **characterized in that** conditioning is realized by intravenous or intraperitoneal administration of the cell culture supernatant.

5. Method according to one of the claims 1 to 4, **characterized in that** conditioning is carried out in a period of 4 weeks after transplantation, beginning with the day of transplantation.

6. Method according to one of the claims 1 to 5, **characterized in that**, in addition to conditioning with cell culture supernatant, support cells that have no hematopoietic potential on their own are administered to the mouse.

7. Method according to one of the claims 1 to 6, **characterized in that** the mouse has an age of more than six weeks

## Revendications

1. Procédé de production d'un modèle d'animal pour le système immunitaire humain dans une souris immunodéficiente, comportant les étapes de :
a) transplantation de cellules souches hématopoïétique humaines dans une souris NOD/SCID, et
b) conditionnement de la souris avec un surnageant de culture cellulaire d'une culture de cellules d'une lignée cellulaire humaine sélectionnée parmi 358/8 qui est dérivée de NCI-H358, et de sous-clones dérivés de celles-ci, ainsi que A-549, où le surnageant de culture cellulaire contient respectivement 20 pg/ml à 10 ng/ml des facteurs humains interleukine-8, interleukine-6 et facteur de croissance de l'endothélium vasculaire (VEGF), une protéine 10 induite par l'interféron γ, une protéine-1 chimio-attractive monocytaire, l'angiogénine et un activateur plasminogène de type tissu.

2. Procédé selon la revendication 1, **caractérisé en ce que** les cellules souches humaines avec potentiel hématopoïétique proviennent de moelle osseuse humaine, de sang périphérique mobilisé ou non mobilisé, de sang de cordon ombilical ou de tissu de cordon ombilical.

3. Procédé selon la revendication 1 ou 2, **caractérisé en ce que** la transplantation se produit 3 à 24 heures après une irradiation de la souris.

4. Procédé selon l'une des revendications 1 à 3, **caractérisé en ce que** le conditionnement se produit par application intraveineuse ou intrapéritonéale du surnageant de culture cellulaire.

5. Procédé selon l'une des revendications 1 à 4, **caractérisé en ce que** le conditionnement est réalisé sur une durée de 4 semaines après la transplantation, en commençant au jour de la transplantation.

6. Procédé selon l'une des revendications 1 à 5, **caractérisé en ce qu'**en plus du conditionnement avec le surnageant de culture cellulaire, on réalise une application de cellules de soutien sans potentiel hématopoïétique propre dans la souris.

7. Procédé selon l'une des revendications 1 à 6, **caractérisé en ce que** l'âge de la souris est supérieur à 6 semaines.
